Europäisches Patentamt

European Patent Office

Office européen des brevets

(19)

(11) Veröffentlichungsnummer: **0 035 260**
A2

# EUROPÄISCHE PATENTANMELDUNG

(21) Anmeldenummer: 81101452.1

(22) Anmeldetag: 27.02.81

(51) Int. Cl.³: **G 01 N 33/48**, A 61 B 5/00

(30) Priorität: **29.02.80 DE 3007722**

(71) Anmelder: **Krone, Herbert, Niedernkamp 11, D-4933 Blomberg-Donop (DE)**

(43) Veröffentlichungstag der Anmeldung: **09.09.81 Patentblatt 81/36**

(72) Erfinder: **Krone, Herbert, Niedernkamp 11, D-4933 Blomberg-Donop (DE)**

(84) Benannte Vertragsstaaten: **AT BE CH DE FR GB IT LI LU NL SE**

(74) Vertreter: **Patentanwälte Ter Meer-Müller-Steinmeister, Artur-Ladebeck-Strasse 51, D-4800 Bielefeld 1 (DE)**

(54) Verfahren zur Bestimmung der Gerinnung von Flüssigkeiten und Coagulometer zu dessen Durchführung.

(57) Für ein Verfahren und ein Coagulometer zur Bestimmung des Gerinnungszeitpunktes von Flüssigkeiten, insbesondere Blut ist ein um eine gegen die Senkrechte geneigte Achse drehbarer Behälter (10) vorgesehen, der eine im wesentlichen ringförmige, gegen die Waagerechte geneigte Bahn bildet. Der Behälter ist derart bemessen und geneigt, dass sich die Flüssigkeit vor der Gerinnung in einer Linse im tieferen Bereich (22) des Behälters (10) sammelt. Mit beginnender Gerinnung haftet die Flüssigkeit an dem Boden des Behälters an und wird daher in höhere Bereiche der Umlaufbahn mitgenommen, in denen sie abgetastet werden kann.

EP 0 035 260 A2

0035260

## BESCHREIBUNG

Die Erfindung betrifft ein Verfahren zur Bestimmung der Gerinnung von Flüssigkeiten sowie ein Coagulometer zu dessen Durchführung gemäß dem Oberbegriff des Hauptanspruchs und des ersten Vorrichtungsanspruchs.

Es sind zahlreiche Verfahren zur Gerinnungsbestimmung bzw. zur Messung des Gerinnungszeitpunktes bekannt. Gerinnungsuntersuchungen dienen beispielsweise zur Klärung des Gerinnungsverhaltens von Blut. Für das Gebiet der Erfassung der Blutgerinnung befindet sich eine Übersicht zum Stand der Technik beispielsweise in dem Aufsatz von F.P. Gauper "Blutgerinnung - Methoden zur Erfassung des Gerinnungseintritts" in GIT-Fachzeitschrift für das Laboratorium, 19. Jahrgang, Sonderheft 1975, Seite 509 bis 512.

Die Entwicklungen der letzten Jahre befassen sich insbesondere mit der Möglichkeit einer automatischen Registrierung und Aufzeichnung des Gerinnungszeitpunkts. Bei einigen bekannten Methoden wird eine Kugel oder dgl. in ein gedrehtes oder anderweitig bewegtes Meßgefäßt zur Aufnahme von Blut eingebracht und durch ein magnetisches Feld, durch Schwerkraft etc. in einer vorgegebenen, von der Bewegung des Meßgefäßes unabhängigen Position gehalten. Bei einsetzender Gerinnung und damit Fibrin-Bildung wird die Kugel durch das innerhalb des Meßgefäßes entstehende Fibrin-Gerüst bei den Bewegungen des Meßgefäßes mitgenommen. Der Zeitpunkt, an dem sich die Kugel aus ihrer ursprünglichen Position bewegt, kann optisch, induktiv, kapazitiv etc. abgetastet werden.

Diesen Verfahren ist der Nachteil gemeinsam, daß neben einem zumeist als Wegwerfartikel ausgebildeten Meßgefäß eine Kugel erforderlich ist, die zumeist mit erheblicher Genauigkeit hergestellt werden muß, so daß sie nicht als

Wegwerfartikel in Betracht kommt und daher bei jedem Meßvorgang sorgfältig gereinigt werden muß.

Daneben sind rein optische Methoden bekannt, bei denen die mit der Gerinnung eintretende Trübung durch eine Lichtschranke ermittelt wird. Diese Methoden kommen zwar für Plasma-Analysen, nicht jedoch für Vollblut in Betracht.

Weitere bekannte Verfahren gehen von dem veränderten Schwingungsverhalten einer Probe bei Gerinnungseintritt aus. Entsprechende Geräte haben jedoch den Nachteil, daß sie wegen der erforderlichen hohen Empfindlichkeit anfällig gegenüber äußeren Erschütterungen sind.

Der Erfindung liegt die Aufgabe zugrunde, ein Verfahren und eine Vorrichtung der gattungsgemäßen Art zu schaffen, die in möglichst einfacher und apparativ wenig aufwendiger Weise eine zuverlässige Ermittlung des Gerinnungszeitpunktes gestatten.

Diese Aufgabe wird bei einem Verfahren der eingangs genannten Art dadurch gelöst, daß eine zu prüfende Flüssigkeit in einem gegen die Waagerechte geneigten Ringraum in einer Menge eingebracht wird, die nur den tiefergelegenen Bereich des Ringraumes einnimmt, daß der Ringraum um seine gegen die Senkrechte geneigte Rotationsachse gedreht wird und daß die Mitnahme der Flüssigkeit in höher gelegene Bereiche des Ringraumes abgetastet wird.

Ein derartiges Verfahren ist mit äußerst einfachen Mitteln zuverlässig durchführbar. Als "Ringraum" der genannten Art kommt bereits ein um eine geneigte Achse drehbar, als Wegwerf-Artikel hergestellter Becher in Betracht, dessen Durchmesser derart gewählt und der derart geneigt sein muß, daß das Volumen einer üblichen Probe sich nach

0035260

dem Einfüllen in den Becher in dessen tiefer gelegenem Bereich sammelt, nicht jedoch den gesamten Boden bedeckt. Vorzugsweise ist der Boden jedoch nicht flach, sondern als ringförmige Rinne ausgebildet, durch die die von der Probe eingenommene Fläche exakter abgegrenzt werden kann. Die mit der Flüssigkeitsprobe in Berührung kommende Fläche des Ringraumes kann aufgerauht oder mit Rippen oder Nuten oder dgl. versehen sein.

Bei der Durchführung des erfindunsgemäßen Verfahrens verbleibt die Flüssigkeitsprobe bei der Drehung des Ringraumes aufgrund der auf sie einwirkenden Schwerkraft zunächst stets in dem jeweils tiefstgelegenen Bereiches des Ringraumes. Mit eintretender Gerinnung haften jedoch die sich bildenden Fibrin-Fäden an den Flächen des Ringraumes an, so daß die geronnene Flüssigkeit bei der Drehung des Ringraumes entgegen der Schwerkraft in Umlaufrichtung mitgenommen wird. Eine Meßschranke, die beispielsweise in einem Winkelabstand von 90° in bezug auf den tiefstgelegenen Punkt der Bahn des Ringraumes angeordnet sein kann, gestattet es, die Mitnahme der geronnenen Flüssigkeit bei der Drehung des Ringraumes abzutasten. Ein gewisser Abstand zwischen der Meßschranke und dem tieferen Bereich der Bahn des Ringraumes ist erforderlich, um eine Registrierung leichter Schwingungsbewegungen der Probe bei der Drehung des Ringraumes auszuschalten.

Als Meßschranke kommt in erster Linie eine Lichtschranke in Betracht. Beispielsweise können jedoch auch kapazitive Meßverfahren verschiedener Art verwendet werden.

Zum Antreiben des Probenbehälters in Drehrichtung und zugleich zur Unterbringung der Meßschranke ist der Boden des Behälters vorzugsweise im Mittelbereich durch einen von unten hohlen Mittelzapfen angehoben. Der Mittelzapfen kann einerseits zur Anbringung des Behälters auf der Welle eines Drehantriebs verwendet werden und im übrigen einen Punkt einer Meßschranke aufnehmen.

Der Proben-Behälter kann beispielsweise aus durchsichtigem Kunststoff, ggf. allerdings auch aus Glas bestehen.

Im Folgenden wird ein bevorzugtes Ausführungsbeispiel eines erfindungsgemäßen Coagulometers anhand der beigefügten Zeichnung näher erläutert.

Fig. 1    ist ein schematischer Schnitt durch einen Teilbereich eines erfindungsgemäßen Coagulometers mit einem Proben-Behälter;

Fig. 2    zeigt eine Draufsicht in Richtung der Pfeile 2-2 in Fig. 1;

Fig. 3    entspricht Fig. 1, zeigt jedoch eine weitere Ausführungsform;

Fig. 4    ist eine Draufsicht auf Fig. 3.

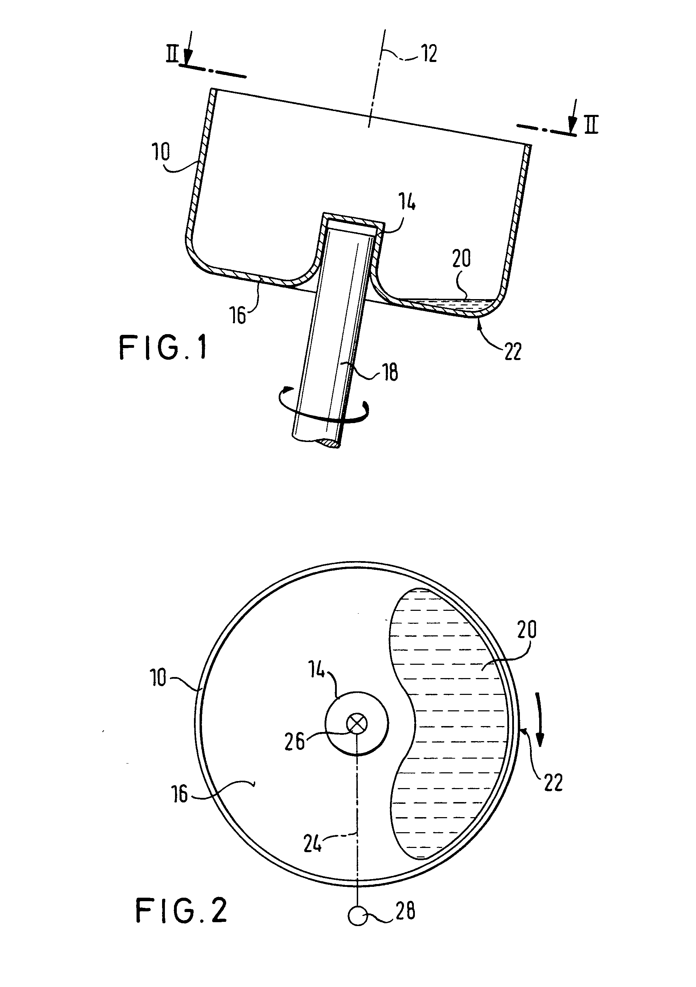

In Fig. 1 ist im Querschnitt ein insgesamt zylindrischer Proben-Behälter 10 gezeigt, dessen Rotationsachse 12 um etwa 5-25$^b$ gegen die Senkrechte geneigt ist. Im dargestellten Beispiel weist der Proben-Behälter 10 einen angehobenen, von unten hohlen Mittelzapfen 14 am Boden 16 auf, der unter anderem dazu dient, den Proben-Behälter 10 auf einen drehbar angetriebenen Wellenstumpf 18 aufzusetzen, dessen Antrieb in der Zeichnung nicht gezeigt ist.

Der Durchmesser des Proben-Behälters 10 ist erheblich grösser als derjenige üblicher Meßröhrchen, so daß in Verbindung mit der Neigung der Rotationsachse 12 die Möglichkeit besteht, eine mit 20 bezeichnete Blutprobe im tieferen Bereich 22 der Umlaufbahn des Proben-Behälters 10 unterzubringen, ohne daß die Blutprobe den gesamten Boden 16 des Behälters bedeckt. Die von der Blutprobe 20 eingenommene Fläche ist in Fig. 2 genauer erkennbar.

Während die Blutprobe 20 bei ausreichend fließfähigem Zustand des Blutes aufgrund der Schwerkraft auch während der Drehung des Proben-Behälters 10 im tieferen Bereich 22 der Umlaufbahn des Behälters verbleibt, haftet das gerinnende Blut an der Innenfläche des Bodens 16 an, so daß es bei der Drehung des Proben-Behälters 10 entgegen der Schwerkraft mitgenommen wird. Sobald das anhaftende, geronnene Blut die Höhe einer in Fig. 2 mit 24 bezeichneten Meßschranke erreicht, gelangt ein Impuls an nachgeschaltere Registrierungs- und Steuergeräte, die im einzelnen bekannt sind und hier nicht näher erläutert werden sollen.

Sofern als Meßschranke eine Lichtschranke verwendet wird, kann ein Punkt der Meßschranke, beispielsweise eine Lichtquelle 26 im Inneren des in diesem Falle hohl ausgeführten Wellenstumpfes 18 untergebracht werden, während sich eine zugehörige Fotozelle 28 außerhalb des Proben-Behälters 10 befindet. Zu diesem Zweck muß der Wellenstumpf 18 zumindest in seinem oberen Endbereich durchsichtig ausgebildet sein oder zumindest ein entsprechendes, umlaufendes Fenster aufweisen.

Im übrigen kann die Abtastung beispielsweise auch kapazitiv von der Außenseite des Proben-Behälters 10 erfolgen.

Eine weitere Abtastmöglichkeit besteht darin, im tieferen Bereich 22 der Umlaufbahn des Proben-Behälters eine auf die Blutprobe 20 einwirkende niederfrequente Schwingung aufzubringen. Ein Empfänger für eine derartige Schwingung kann in diesem Falle wiederum im Winkel von etwa 90° versetzt angeordnet sein. Sobald das Blut gerinnt und am Boden des Proben-Behälters 10 anhaftet, überträgt sich die niederfrequente Schwingung vom tieferen Bereich 22 aus über die Bahn des anhaftenden Blutes bis zum dem Bereich des Empfängers. Bei geeigneter Eichung des Empfängers und der nachgeschalteten Elektronik besteht die Möglichkeit, nicht nur den Beginn der Gerinnung als solchen abzutasten, sondern quantitative Aussagen in der Form einer sogenannten Gewinnungskurve über die Geschwindigkeit des Gerinnungsvorgangs zu gewinnen.

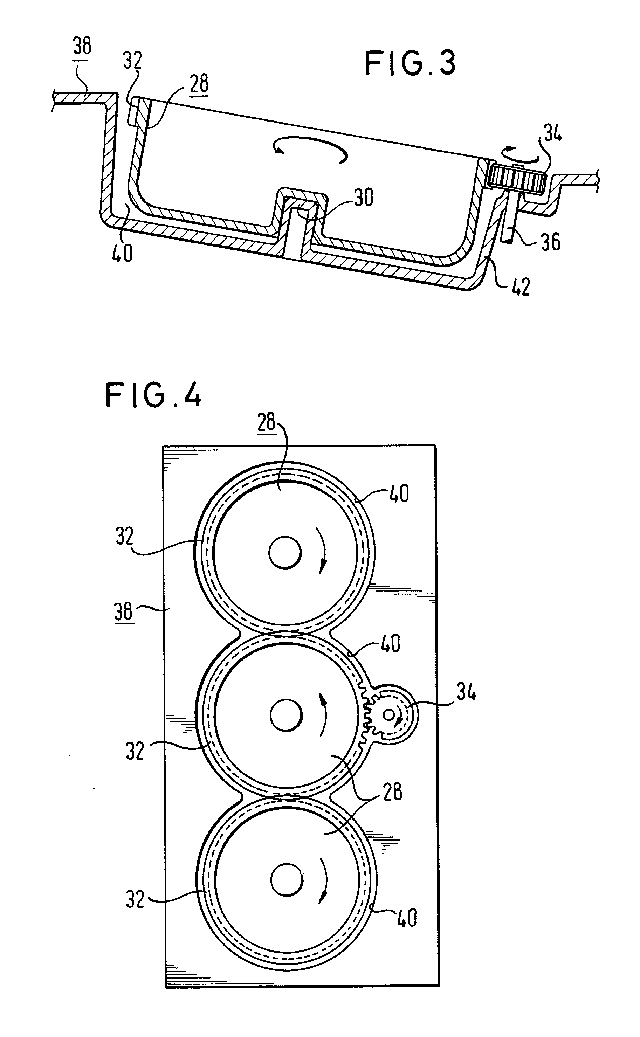

Da der Behälter als kostengünstig herzustellender Wegwerfartikel ausgeführt werden kann, bietet sich eine weitere Möglichkeit, die insbesondere bei der gleichzeitigen Untersuchung mehrerer Proben in Laboratorien oder dgl. von Bedeutung sein kann. In diesem Falle kann anstelle des drehbaren Wellenstumpfes 18 eine entsprechende, feststehende Achse vorgesehen sein, deren Oberfläche derart auszubilden ist, daß der aufgesetzte Behälter mit verhältnismäßig geringer Reibung drehbar ist. Diese Ausführungsform der Erfindung ist in Fig. 3 und 4 veranschaulicht.

Der in diesem Falle mit 28 bezeichnete Behälter ist auf einer feststehenden, ebenfalls leicht geneigten Achse 30 angeordnet und auf dieser verhältnismäßig leicht drehbar. Auf dem äußeren Umfang des Behälters befindet sich ein Zahnkranz 32, mit dem ein Kegelritzel 34 kämmt, das drehfest auf einer aus einem Gehäuse 38 in senkrechter Richtung austretenden, drehbaren Welle 36 angeordnet ist.

Da der Behälter 28 und dessen Zahnkranz 32 ein Einwegartikel ist, erfordert der Zahnradeingriff keine hohe Genauigkeit; die etwa bei einer Berechnung auf lange Lebensdauer in einem ständig verwendeten Getriebe notwendig wäre. Auch eine Umlenkung der Antriebskräfte aufgrund der zwangsläufig geneigten Stellung des Behälters erfordert kein hochwertiges Umlenkgetriebe, so daß beispielsweise ohne weiteres ein Übergang von der Kegelverzahnung des Kegelritzels 34 zu einer einfachen Stirnverzahnung des Zahnkranzes 32 auf dem Umfang des Behälters möglich ist.

Der in Fig. 3 gezeigte Mechanismus kann ohne weiteres und insbesondere ohne zusätzliche Verzweigungsgetriebe verwendet werden, wenn mehrere Proben gleichzeitig untersucht werden sollen. In diesem Falle müssen lediglich mehrere Proben gleichzeitig untersucht werden sollen. In diesem Falle müssen lediglich mehrere feststehende Achsen 30 auf der Oberseite des Gehäuses 38 vorgesehen sein, deren Abstände und Positionen derart bemessen sind, daß die Zahnkränze 32 der aufgesetzten Behälter 28 miteinander kämmen. Jeder zusätzliche Behälter wird dabei durch den bereits durch das Kegelritzel 34 angetriebenen Behälter mitgenommen. Die sich jeweils ändernde Drehrichtung kann ohne weiteres durch entsprechende Anordnung der Meßschranke berücksichtigt werden. Die Reihe der Behälter ist nahezu beliebig fortsetzbar.

Ein entsprechendes, auf Dauerhaftigkeit berechnetes Verzweigungsgetriebe würde einen erheblichen Aufwand bedeuten.

0035260

PATENTANSPRÜCHE

1. Verfahren zur Bestimmung der Gerinnung von Flüssigkeiten, dadurch g e k e n n z e i c h n e t, daß eine zu prüfende Flüssigkeit in einen gegen die Waagerechte geneigten Ringraum in einer Menge eingebracht wird, die nur den tiefergelegenen Bereich des Ringraumes einnimmt, daß der Ringraum um seine gegen die Senkrechte geneigte Rotationsachse gedreht wird, und daß die Mitnahme der Flüssigkeit in höher gelegene Bereiche des Ringraumes abgetastet wird.

2. Verfahren nach Anspruch 1, dadurch g e k e n n z e i c h n e t, daß die Mitnahme der Flüssigkeit optisch abgetastet wird.

3. Verfahren nach Anspruch 1, dadurch g e k e n n z e i c h n e t, daß die Mitnahme der Flüssigkeit kapazitiv abgetastet wird.

4. Coagulometer zur Durchführung des Verfahrens gemäß einem der Ansprüche 1 bis 3, mit einem zylindrischen, um seine gegen die Senkrechte geneigte Achse drehbaren Proben-Behälter und einer radial zu dem Proben-Behälter angeordneten Meßschranke, dadurch g e k e n n z e i c h n e t, daß der Proben-Behälter (10) derart bemessen und geneigt ist, daß er in seinem tiefer gelegenen Bereich (22) eine für die Gerinnungsmessung ausreichende Flüssigkeitsmenge aufnimmt.

5. Coagulometer nach Anspruch 4, dadurch g e k e n n z e i c h n e t, daß der Proben-Behälter (10) becherförmig mit einer im Boden vorgesehen, vertieften, ringförmigen Rinne ausgebildet ist.

6. Coagulometer nach Anspruch 4, dadurch g e k e n n z e i c h n e t, daß der Proben-Behälter becherförmig mit einem in der Rotationsachse (12) angehobenen, von unten hohlen Mittelzapfen (14) ausgebildet ist.

7. Coagulometer nach einem der Ansprüche 4 bis 6, dadurch g e k e n n z e i c h n e t, daß der Proben-Behälter eine rauhe oder geriffelte innere Oberfläche aufweist.

8. Coagulometer nach einem der Ansprüche 4 bis 7, dadurch g e k e n n z e i c h n e t, daß die Rotationsachse (12) des Proben-Behälters (10) um 5-25° gegen die Senkrechte geneigt ist.

9. Coagulometer nach einem der Ansprüche 6 bis 8, dadurch g e k e n n z e i c h n e t, daß ein Punkt einer Meßschranke (24) im Inneren des in den hohlen Mittelzapfen (14) eintretenden Endes eines den Proben-Behälter (10) in Drehrichtung antreibenden Wellenstumpfes (18) angeordnet ist.

10. Coagulometer nach einem der Ansprüche 6 bis 8, dadurch g e k e n n z e i c h n e t, daß auf dem äußeren Umfang des Proben-Behälters (28) ein Zahnkranz (32) ausgebildet ist.

II

12

10

14

20

16

18

**FIG.1**

22

10

20

14

26

16

22

24

28

**FIG.2**

# FIG.3

# FIG.4